# EUROPEAN PATENT APPLICATION

(11) **EP 4 057 293 A1**
(43) Date of publication of application: **14.09.2022**
(21) Application number: 21161948.1
(22) Date of filing: 11.03.2021
(51) Int. Cl.: G16H 40/20

(54) **METHOD AND CONTROLLER FOR SIGNALLING A CARE NEED TO A USER**

(71) Applicant: AssistMe GmbH, 10555 Berlin (DE)
(72) Inventor: Carucci Lemke, Diego, 10245 Berlin (DE); Grimmer, Silke, 10179 Berlin (DE)
(74) Representative: Gulde & Partner

(57) **Abstract**

The invention relates to a method for signalling a care need, in particular a high priority care need of a subject (1), to a user (2), the method being of a controller (30) of an electronic device. A first step (S1) of the method is obtaining sensor information (i2) and first location information (i3) related to at least one subject (1), the sensor information (i2) being indicative of a care need of the at least one subject (1). In a second step (S2) second location information related to at least one user (2) are obtained. In a next step (S3) at least one subject (1) in need of care based on the sensor information (i2) is determined and a further step (S4) comprises generating output information (i1) for one of the at least one user (2), the output information (i1) being based on the care needs and the first location information (i3) of the at least one subject (1) and on the second location information. Another aspect of the invention is a system comprising a controller (30) configured to perform the method, at least one wearable sensor (10), at least one device (20) and localisation means (40). The invention further relates to a controller (30) that is configured to perform the inventive method and to a device (20) comprising such a controller.

## Description

The invention relates to a method for signalling a care need, in particular a high priority care need, to a user by a controller of an electronic device. Further it relates to a system comprising a controller configured to perform the method, at least one wearable sensor, at least one handheld device and localisation means. The invention further relates to such a controller itself, to a device comprising such controller and to a software program for operating such controller.

One of the biggest challenges that modern societies have to face in coming years is the aging of their populations. The number of caregivers and elderly at nursing homes will become less balanced, therefore less caregivers will attend more residents. Already today caregivers do check on residents according to a tight time schedule, not only making it an exhausting workload but also rendering it difficult to figure out, which urgent need to attend first. Also important to note is that caregivers have assigned a certain number of residents they have to monitor on each shift, which renders efficient scheduling even more important.

Incontinence is a condition in which there is uncontrolled release of natural discharges or evacuations from the bladder and/or bowel. Urinary incontinence refers to loss of bladder control resulting in involuntary or uncontrolled urination. While some forms of incontinence, particularly urinary/bladder incontinence are relatively widespread, the condition typically affects the elderly and the infirm and is more prevalent among women.

In the past, to comply with regulations and protocols and to ensure that incontinence sufferers in care institutions such as hospitals, nursing homes, aged care facilities and geriatric institutions are appropriately cared for, it has been necessary for staff to manually check these patients on a regular basis. Apart from the unpleasantness involved with manual checks, such a regimen also places a strain on staff resources. Manually checking for wetness can also cause interruption to a patient's rest and sleep.

Another current pain point at care homes are night shifts, wherein one of the main challenges is the wide area of the care home, and the reduced numbers of caregivers during these shifts. Therefore the physical strain on caregivers becomes a problem.

It is thus an object of the present disclosure to overcome or reduce at least some of the drawbacks of the prior art and to provide a method for signalling a care need, in particular a high priority care need, to a user.

The given task is solved by the subject-matter of the independent claims 1 and 12. Advantageous embodiments of the invention can further be gained from the dependent claims.

A first aspect of the invention refers to a method for signalling a care need to a user by a controller of an electronic device. A care need, in the sense of the present disclosure, is a condition of a human being that momentarily requires assistance and/or care. Exemplarily, a care need is an acute condition of a human being that can be improved by an action that cannot be performed by the human being himself or herself, such as, for example, a stool or urine occurrence in an incontinence material that can be improved by replacing the material. A user, in the sense of the present disclosure, is a human being who is entrusted with the care of at least one other human being. Preferably, a user is trained to treat a care need. For example, a user is a nurse, a nurse practitioner, a nurse for the elderly, a physician, a doctor, and/or other caregiver. Alternatively preferred, a user is a robot configured to tread a care need.

The method comprises the step of obtaining sensor information and first location information related to at least one subject. A subject, in the sense of the present disclosure, is a human being that regularly requires assistance and/or care, such as a patient of a hospital or a resident of a nursing or care home. The sensor information are indicative of a care need of the at least one subject. In other words, a possible care need of the at least one subject can preferably be determined on the basis of the sensor information. The first location information preferably relates to a first zone in which the at least one subject is located or to a first position where the at least one subject is located. The first zone is preferably a room, a corridor, a floor and/or a station in which the at least one subject is located. The first position is preferably the geographical position of the at least one subject. Preferably, sensor information and first location information related to one subject are obtained. Further preferred, a plurality of sensor information and first location information each one of the plurality being related to one of a plurality of subjects respectively is obtained.

In a further step of the method, second location information related to at least one user are obtained. The second location information preferably relates to a second zone in which the at least one user is located or to a second position where the at least one user is located. The second zone is preferably a room, a corridor, a floor and/or a station in which the at least one user is located. The second position is preferably the geographical position of the at least one user. Preferably, second location information related to one user are obtained. Further preferred, a plurality of second location information each one of the plurality being related to one of a plurality of users is obtained.

Another step of the method comprises determining at least one subject in need of care based on the sensor information. A subject in need of care, in the sense of this disclosure, is exemplarily a subject in an acute condition that requires momentary assistance and/or care from a user, i.e., within a certain, e.g., predefined, time. Preferably, the sensor information of the at least one subject is evaluated and the at least one subject is determined to be in need of care if the sensor information identifies a care need. Exemplarily, the evaluation is based on comparing at least one sensor signal with a predetermined threshold. However, also definitions of care needs based on a combination of multiple sensor signals is preferred.

A further step of the method comprises generating of output information for one of the at least one user. In other words, output information intended for one of the at least one user is generated preferentially. The output information is preferably generated so that it can be output to the one user. The output information is preferably based on the care needs and the first location information of the at least one subject in need of care and on the second location information. Preferably, both the content and the recipient, in other words the one user, of the output information are based on the care needs and the first location information of the at least one subject in need of care and on the second location information. Preferably, the output information includes the care need determined for the at least one subject and the first position and/or first zone based on the first location information of the at least one subject in need of care. Further preferably, the output information additionally includes personal information about the at least one subject in need of care, such as his or her name.

Further preferred, the output information also includes the first position and/or first zone based on the first location information and personal information of at least one subject determined to be not in a momentary need of care. Preferably, the information about the at least one subject in need of care is emphasized over that about the at least one subject not determined to be in need of care in case that the output information includes both. Especially preferred, the steps of the method according to the disclosure are repeated periodically and thus the output information is regularly adapted to the current situation.

By means of the method according to the disclosure, care needs of one or more subjects can advantageously be recognized automatically and transmitted to one or more users. Therefore, tactile, and visual checks at the subjects can be avoided and the time spent for the actual care, for example the time spend for changing incontinence products, can be reduced which significantly increases the comfort of the subjects. The method can advantageously be applied both in a personal care relationship and in a nursing home with a plurality of subjects and users. The caregivers benefit from the method by being notified whenever there is a care need of one subject and the plurality of users in the nursing home benefit from the method by regularly learning which of the plurality of subjects are currently in need of assistance.

According to a preferred embodiment of the method according to the disclosure, sensor information and first location information of a plurality of subjects are obtained. Preferably a plurality of sensor information and first location information are obtained, each one of the plurality of information being related to one of the plurality of subjects respectively. The output information in this preferred embodiment preferably relates to one of the at least one subject that is determined having a shortest distance to the one user and/or a highest care need. The distances between each of the plurality of subjects and the one user are preferably determined based on the first location information and the second location information. The highest care need is preferably the care need that requires the fastest intervention from the one user.

Further preferably, the one of the at least one subject is determined additionally based on a responsibility of the one user. In other words, the one user is further responsible for the one of the at least one subject, for example, is the responsible nurse for him or her. Additionally preferred, the one of the at least one subject is determined further based on a timer indicating a time since the one subject was lastly visited by a user. Preferably the one of the at least one subject is determined further having a greatest time since he or she was lastly visited by a user.

Preferably, the output information includes the care need determined for the one of the at least one subject and the first position and/or first zone based on the first location information of the one of the at least one subject. Further preferably, the output information additionally includes personal information about the one of the at least one subject, such as his or her name. In some cases, the output information may also include information about others of the plurality of subjects. In such case, the information about the one of the at least one subject is emphasized over that about the other subjects.

According to another preferred embodiment, second location information of a plurality of users are obtained. Preferably, a plurality of second location information is obtained, each one of the plurality of information being related to one of the plurality of users respectively. The one user in this preferred embodiment is preferably determined based on a distance to the at least one subject, qualification information and/or capacity information of the plurality of users. The distances between the at least one subject and each of the plurality of users are preferably determined based on the first location information and the second location information. The one user is preferably determined as having the shortest distance to the at least one subject.

The qualification information relates preferably to a qualification of a user to treat a care need and is preferably determined based on a user profile. The one user is preferably determined as having a qualification information relating to a qualification to treat the care need of the at least one subject. The capacity information preferably relates to a workload of a user. The capacity information is preferably determined based on a number of output information generated for the user, based on a timer indicating the time since the last visit of a subject and/or based on a user input. The one user is preferably determined as having the highest capacity. Further preferably, the one user is determined additionally based on a responsibility of the one user for the at least one subject. In other words, the one user is further determined as being responsible for the at least one subject, for example, being the responsible nurse for the at least one subject.

In the two preferred embodiments described above, the output information is generated advantageously on the basis of a prioritized subject and a prioritized user, respectively. In one case, in a user-centered approach, one subject is selected from a plurality of subjects with regard to the one user and the content of the output information is adapted thereto. In the other case, in a subject-centered approach, the one user is selected from a plurality of users with regard to the at least one subject and thus the recipient of the output information is adapted. Both approaches advantageously lead to an optimization of the care process, since the output information is created in such a way that it conveys the subject with the most urgent care need to the most appropriate user. In this way, for example, it is possible to bring to a caregiver's attention an urgent need that is closest to his or her location, avoiding other caregivers to have to move from farther locations inside a care home. Particularly preferred, these two approaches are combined, i.e., output information for a subject most in need of care is generated for one user most capable of taking care of the subject with certain care quality and/or in timely manner.

A further preferred embodiment comprises the additional step of generating a plurality of care information based on the care needs and the first location information of the plurality of subjects. Preferably, care information for each of the plurality of subjects are generated, each of the care information comprising the care need and first location information of the respective subject. The care information preferably further comprise personal information of the respective subject, such as the subject's name. As a further step, the preferred embodiment comprises a prioritizing of the plurality of care information based on the care needs, wherein the highest priority care information indicate the highest care need. Preferably, a care need that requires rapid assistance is indicative of high priority care information. Further preferred, the prioritization of a care need is read out from a look up table, LUT, or a search tree. In the LUT or the search tree, the care needs are preferably ordered according to their priority or stored with an associated priority number that indicates the level of their prioritization. The order of the care needs or the priority number are preferably adjustable by the user. This preferred embodiment provides the user with a sequence of subjects with care needs, starting with the most urgent care need and ending with the least urgent care need, which advantageously leads to a further optimization of the care process.

Another preferred embodiment further comprises the step of generating and prioritizing a plurality of user information based on the qualification information, the capacity information and/or the second location information of the plurality of users. Preferably, user information for each of the plurality of users are generated, each of the user information comprising the qualification information, the capacity information and/or the second location information of the respective user. The one user preferably has the highest priority user information. In other words, the output information is generated for the one user with the highest priority user information.

In a further preferred embodiment, a qualification information indicating a qualification for a plurality of care needs, a capacity information indicating a high capacity and/or a second location information indicating a short distance to the at least one subject determines a high priority user information.

According to a further preferred embodiment, the second location information indicate predicted locations of the plurality of users. In other words, the second location information further indicate a predicted location for each user of the plurality of users at a prospective point in time. The second location information preferably relate to third zones in which each user of the plurality of users will be located respectively or to third positions where each user of the plurality of users will be located respectively. The third zones are preferably rooms, corridors, floors and/or stations in which each user of the plurality of users will be located respectively. The third positions are preferably geographic positions of each user of the plurality of users.

In the preferred embodiment, the method according to the disclosure advantageously further allows prioritizing the tasks of the users for subject treatments based on predictions of their soon to be locations. For example, if a user is currently on the stairs from the first to the second floor, the second location information indicate that the user will soon be on the second floor, and subjects in need of care on the second floor are provided to him prioritized by the output information. This advantageously leads to a further optimization of the care flow.

In another preferred embodiment of the method, the sensor information comprises at least one of a temperature, a humidity, an acceleration, a gas presence, an orientation and/or a sound level. Preferably, the care need of the at least one subject is determined on the basis of at least one, preferably multiple values of the sensor information. Further preferred, the care need of the at least one subject is determined based on at least one, preferably multiple values of the sensor information exceeding predetermined trigger values. The trigger values are preferably stored in and read out from a LUT or a search tree.

Preferably, a filled incontinence material is determined as the care need when the humidity exceeds a predetermined trigger value. Further preferably, if in addition to the humidity, further the temperature and/or the gas presence exceed respective predetermined trigger values, urine or stool in the incontinence material, a completely filled incontinence material (absorption before leakage, ABL) or leakage is determined as the care need. If the acceleration and/or the orientation exceed respective predetermined trigger values, a non sleep movement is preferably determined as the care need. Further, the acceleration and/or the orientation exceeding predefined trigger values preferably leads to a determination of an exit of a secure area as the care need preferably further based on the first location information. Further preferred, if the acceleration exceeds a predefined trigger value, the care need is determined as a fall. If the sound level exceeds a predefined trigger value, it indicates a possible cry for help by the at least one subject, preferably leading to an emergency situation being determined as the care need.

A further aspect of the invention is a system for signalling a care need to a user. The system preferably comprises a controller configured to perform a method as described above. The controller is preferably established in at least one electronic device. Further preferred the controller is established in a plurality of electronic devices wherein each of the electronic devices is configured to perform a part of the controller's functions. Further preferably, the controller or a part of the controller is established in a cloud server, wherein a cloud server is a server of a network.

The system preferably further comprises at least one wearable sensor associated with at least one subject. Preferably, the system comprises one wearable sensor worn by one subject.

Particularly preferably, the system comprises a plurality of wearable sensors worn by a plurality of subjects, each subject of the plurality of subjects wearing one wearable sensor from the plurality of sensors. Preferably, a wearable sensor is attached to an incontinence material worn by a subject. The at least one wearable sensor is preferably configured to detect first location information of the at least one subject and sensor information indicating a care need of the at least one subject. Preferably the at least one wearable sensor is configured to detect sensor data, determine the first location information and the sensor information based on the sensor data and send the first location information and the sensor information to the controller. Alternatively preferred, the at least one wearable sensor is configured to detect the sensor data and transmit the sensor data to an edge computer, wherein the edge computer functions as security gate and pre-processing unit and is configured to determine the first location information and the sensor information based on the sensor data and send the first location information and the sensor information to the controller. Alternatively preferred, the at least one wearable sensor is configured to detect the sensor data and transmit the sensor data to the controller, wherein the controller is further configured to determine the first location information and the sensor information based on the sensor data.

The system according to the disclosure further comprises at least one device associated with at least one user. Preferably, the system comprises one device worn or hold by one user. Particularly preferably, the system comprises a plurality of devices worn or hold by a plurality of users, each user of the plurality of users wearing or holding one device from the plurality of devices. The at least one device is preferably a handheld device such as, for example, a smartphone or a tablet, further preferred an augmented reality, AR, device such as, for example, AR glasses, google glasses or an AR-enabled smartphone or tablet, or further preferred a wearable device such as, for example, a pager. Particularly preferred, the at least one device further comprises output means configured to display the output information to the at least one user. The controller is preferably further designed to provide the output information comprising care needs to the at least one user by way of the output means. Preferably, the controller provides the output information in such a way that a prioritization of the care needs is clearly recognizable, for example by means of a colour marking or an arrangement of the care needs.

Preferably the system further comprises localisation means configured to perform a localisation of the at least one device. The localisation means preferably comprise a plurality of nodes that are configured to send signals to and/or receive signals from the at least one device to perform the localisation. The signals are preferably wired or wireless communication signals, particularly preferred Bluetooth low energy, BLE, signals. The plurality of nodes is preferably evenly distributed at a place of use of the system according to the disclosure, such as, for example, a nursing home. Estimated node locations are preferably arranged in a predetermined geometric pattern based on a number of nodes in the place of use, wherein the number of nodes is based on the size of the place of use. The controller is preferably configured to determine location information of the at least one device based on the localisation, wherein the at least one device is preferably worn or held by the at least one user and thus the location information corresponds to the second location information.

According to a preferred embodiment of the system according to the disclosure, the controller is established in a cloud server. The localisation means preferably comprise a plurality of transmitters configured to communicate with the at least one device and the cloud server preferably via BLE signals. In other words, in this preferred embodiment, the nodes are formed as transmitters. The at least one device is preferably further configured to send localisation signals indicative of a location of the at least one device to the plurality of transmitters. Preferably, the at least one device sends localisation signals which are received by at least one transmitter from the plurality of transmitters which is within a range of the localisation signals. The localisation signals preferably contain first identification data of the at least one device. The cloud server is preferably configured to receive data indicative of the localisation signals from the transmitters and to determine location information of the at least one device based on the data. The data preferably contain the first identification data, second identification data of the transmitters and signal strengths of the localisation signals. As the at least one device is preferably worn or held by the at least one user, the location information corresponds to the second location information.

Preferably, the controller is configured to determine the locations of the transmitters based on the second identification data in combination with a location plan of the transmitters. The controller is preferably further configured to determine distances of the at least one device to the transmitters based on the strengths of the localisation signals. Particularly preferred, the controller is further configured to determine the second location information based on the locations of the transmitters and distances from the at least one device to the transmitters. Further preferred the controller is further configured to determine the second location information based on a location of at least three transmitters and distances from the at least one device to the at least three transmitters using trilateration. Preferably, the controller is configured to determine the second location information further based on a model of the place of use of the system containing radio frequency propagation information. In this way, inaccuracies caused by weakening of the localisation signals, e.g. by walls, can advantageously be detected and compensated for.

According to an alternatively preferred embodiment of the system, the controller is established in the at least one device. Alternatively preferred, a part of the controller is established in the at least one device and a further part of the controller is established in the cloud server. The localisation means preferably comprise a plurality of beacons configured to send beacon signals to the at least one device. In other words, in this preferred embodiment, the nodes are formed as beacons. The beacon signals are preferably wired or wireless communication signals, particularly preferred BLE signals. A beacon signal preferably contains identification data and/or location data of the sending beacon. Preferably, the controller in the at least one device is further configured to receive the beacon signals and to determine location information of the at least one device based on the beacon signals. As the at least one device is preferably worn or held by the at least one user, the location information correspond to the second location information.

The controller is preferably configured to determine a location of a beacon based on a received beacon signal that was send by the beacon. Preferably the controller is configured to determine the location of the beacon based on the location data and/or the identification data in combination with a location plan of the plurality of beacons. The controller is preferably further configured to determine a distance to a beacon based on a strength of a received beacon signal that was send by the beacon. Particularly preferred, the controller is further configured to determine the second location information based on a determined location of and distance to at least one of the plurality of beacons. Further preferred the controller is further configured to determine the second location information based on a determined location of and distance to at least three beacons using trilateration. Further preferably, the controller is configured to determine second location information indicating a predicted location of the at least one user, based on a sequence of received beacon signals and a location map of the plurality of beacons. Preferably, the controller is configured to determine the second location information further based on a model of the place of use of the system containing radio frequency propagation information. In this way, inaccuracies caused by weakening of the beacon signals, e.g. by walls, can advantageously be detected and compensated for.

Alternatively preferred, the controller is configured to determine the second location information as a zone in which the at least one device is located based on received beacon signals. For this purpose, the controller additionally utilizes a location plan that divides the place of use into different zones and determines which beacons are located in which zones. Based on the signal strength of the beacon signals, the controller determines a proximity of the beacons from the at least one device. The proximity preferably can have four different values: Immediate, Nearby, Far and Unknown. Immediate preferably indicates that the beacon is within a few centimeters of the device. Nearby preferably indicates that the beacon is within a few meters of the device. A "Far" proximity preferably indicates that the beacon is more than a few meters from the device and an "Unknown" proximity indicates that no usable data for the beacon has been received. A zone in which the at least one device is located is preferably determined if the beacon signals of the beacons located in the zone are received and if the signal strengths of these beacon signals indicate a "Nearby" or "Immediate" proximity. This leads to the advantage that no exact distances have to be determined from the signal strengths, which leads to a simplification and quicker determination of the second location information.

In a further preferred embodiment of the system, the at least one wearable sensor is configured to detect at least one of a temperature, a humidity, an acceleration, a gas presence, an orientation and/or a sound level. Preferably, the sensor information of the at least one subject are determined on the basis of at least one, preferably multiple values detected by the wearable sensor.

Another aspect of the invention relates to a controller, preferably a controller of an electronic device, for signalling a care need to a user. The controller is preferably configured to perform the method according to the invention as described above.

A further aspect of the invention relates to a device for signalling a care need to a user. The device is preferably configured to be hold or worn by the user. The device is preferably a handheld device such as, for example, a smartphone or a tablet, further preferred an augmented reality, AR, device such as, for example, AR glasses, google glasses or an AR-enabled smartphone or tablet, or further preferred a wearable device such as, for example, a pager. Preferably, the device comprises a controller configured to perform the method according to the invention as described above. Particularly preferred, the device further comprises output means configured to display the output information to at least one user. The controller is preferably further designed to provide the output information comprising care needs to the user by way of the output means. Preferably, the controller provides the output information in such a way that a prioritization of the care needs is clearly recognizable, for example by means of a colour marking or an arrangement of the care needs.

Another aspect of the invention relates to a computer program comprising instructions which, when the program is executed by a computer, such as a controller of an electronic device, cause the computer to execute the method according to the invention as described above.

Such computer programs are typically executed by utilizing the computing resources in a computing device. Applications are stored on a non-transitory medium. An example of a non-transitory medium is a non-volatile memory, for example a flash memory while an example of a volatile memory is RAM. The computer instructions are executed by a processor. These memories are exemplary recording media for storing computer programs comprising computer-executable instructions performing all the steps of the computer-implemented method according the technical concept presented herein.

Another aspect of the invention relates to a computer-readable storage medium comprising instructions which, when executed by a computer, such as a controller of an electronic device, cause the computer to execute the method according to the invention as described above.

Also, a person of skill in the art should recognize that the functionality of various computing devices may be combined or integrated into a single computing device, or the functionality of a particular computing device may be distributed across one or more other computing devices without departing from the scope of the embodiments of the present invention.

The different embodiments of the invention described herein may be advantageously combined unless the contrary is indicated herein.

Reference is now made to the following figures, in order to describe preferred embodiments of the invention in more detail.
- Figure 1: illustrates a method for signalling a care need to a user by a controller of an electronic device according to a preferred embodiment of the disclosure;
- Figures 2a to 2c: illustrate the generation of output information during various exemplary embodiments of the method according to the disclosure;
- Figures 3a/3b: illustrate a system for signalling a care need to a user according to preferred embodiments of the disclosure;
- Figure 4: illustrates an exemplary environment in which the method according to a preferred embodiment of the disclosure is applied;
- Figure 5: illustrates an exemplary use case of the method according to a preferred embodiment of the disclosure; and
- Figure 6a/6b: illustrate another exemplary use case of the method according to a preferred embodiment of the disclosure.

Some portions of the detailed description which follows are presented in terms of data processing procedures, steps or other symbolic representations of operations on data bits that can be performed on computer memory. Therefore, a computer executes such logical steps thus requiring physical manipulations of physical quantities.

Usually these quantities take the form of electrical or magnetic signals capable of being stored, transferred, combined, compared, and otherwise manipulated in a computer system. For reasons of common usage, these signals are referred to as bits, packets, messages, values, elements, symbols, characters, terms, numbers, or the like.

Additionally, all of these and similar terms are to be associated with the appropriate physical quantities and are merely convenient labels applied to these quantities. Terms such as "processing" or "creating" or "transferring" or "executing" or "determining" or "detecting" or "obtaining" or "selecting" or "calculating" or "generating" or the like, refer to the action and processes of a computer system that manipulates and transforms data represented as physical (electronic) quantities within the computer's registers and memories into other data similarly represented as physical quantities within the memories or registers or other such information storage.

As utilized herein, the term "example" means serving as a non-limiting example, instance, or illustration. As utilized herein, the terms "for example" and "e.g." introduce a list of one or more non-limiting examples, instances, or illustrations.

As used herein, the term "and/or" includes any and all combinations of one or more of the associated listed items. Further, the use of "may" when describing embodiments of the present disclosure refers to "one or more embodiments of the present disclosure." Further, in the following description of embodiments, the terms of a singular form may include plural forms unless the presented context clearly indicates otherwise.

It will be understood that although the terms "first" and "second" are used to describe various elements, these elements should not be limited by these terms. These terms are only used to distinguish one element from another element. For example, a first element may be named a second element and, similarly, a second element may be named a first element, without departing from the scope of the present invention. As used herein, the term "and/or" includes any and all combinations of one or more of the associated listed items and expressions such as "at least one of" when preceding a list of elements, modify the entire list of elements.

Reference will now be made in detail to embodiments which are illustrated in the drawings. Effects and features of the exemplary embodiments will be described with reference to the accompanying drawings. Therein, like reference numerals denote like elements, and redundant descriptions are omitted. The present invention, however, may be embodied in various different forms, and should not be construed as being limited to only the illustrated embodiments herein. Rather, these embodiments are provided solely as examples for fully conveying the aspects and features of the present invention to those skilled in the art.

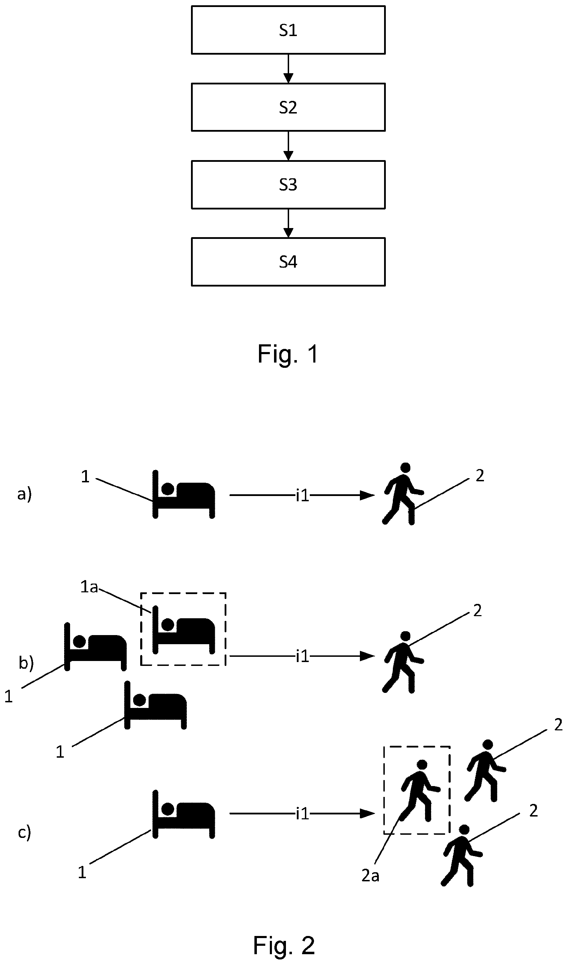

Figure 1 illustrates a method for signalling a care need to a user by a controller of an electronic device according to a preferred embodiment of the disclosure. The method comprises as a first step S1 an obtaining of sensor information and first location information related to at least one subject. The obtained sensor information are indicative of a care need of the at least one subject, so that a possible care need of the at least one subject can be determined on the basis of the sensor information. The first location information relate to a room, a corridor, a floor, a station and/or an exact location in which the at least one subject is located.

In a second step S2 of the method, second location information related to at least one user are obtained, the second location information relating to a room, a corridor, a floor, a station and/or an exact location in which the at least one user is located.

A third step S3 of the method comprises determining at least one subject in need of care based on the sensor information obtained in the first step S1. For this purpose, the sensor information of the at least one subject is evaluated and the at least one subject is determined to be in need of care if the sensor information identifies a care need.

A forth step S4 of the method comprises generating of output information for one of the at least one user. Both the content and the recipient, in other words the one user, of the output information are based on the care needs and the first location information of the at least one subject in need of care and on the second location information obtained in the second step S2. In particular, the steps of the method are repeated periodically and thus the output information is regularly adapted to the current situation.

Figures 2a to 2c illustrate the generation of output information i1 during various exemplary embodiments of the method according to the disclosure. By means of the method, care needs of one or more subjects 1 can advantageously be recognized automatically and transmitted to one or more users 2 as shown in Figure 2a. The output information i1 in this embodiment are generated based on the subject 1 and for the user 2, as represented by the arrow.

A preferred embodiment with a user-centered approach is illustrated in Figure 2b where a plurality of subjects 1 is shown. One of the plurality of subjects 1, namely the first subject 1a, is determined as having a shortest distance to the one user 2 and a highest care need. The determination of the first subject 1a is represented by the dashed outline. In other words, in this user-centered approach, the first subject 1a is prioritized as the subject 1 to be handled first by the user 2. The output information i1 in this embodiment are generated based on the first subject 1a and for the user 2, as represented by the arrow.

A preferred embodiment with a subject-centered approach is illustrated in Figure 2c where a plurality of users 2 is shown. One of the plurality of users 2, namely the first user 2a, is determined based on a distance to the subject 1, qualification information and capacity information. The determination of the first user 2a is represented by the dashed outline. In other words, in this subject-based approach, the first user 2a was prioritized as being the nearest to the subject 1, the most qualified for treating the care need of subject 1 and having the most time for visiting subject 1. The output information i1 in this embodiment are generated based on the subject 1 and for the first user 2a, as represented by the arrow.

The embodiments shown in Figure 2b and 2c may also be combined advantageously. Both embodiments individually or combined advantageously lead to an optimization of the care process, since the output information i1 is created in such a way that it conveys the subject 1a with the most urgent care need to the most appropriate user 2a. In this way, for example, it is possible to bring to a user's 2 attention an urgent need that is closest to his or her location, avoiding other users to have to move from farther locations inside a care home.

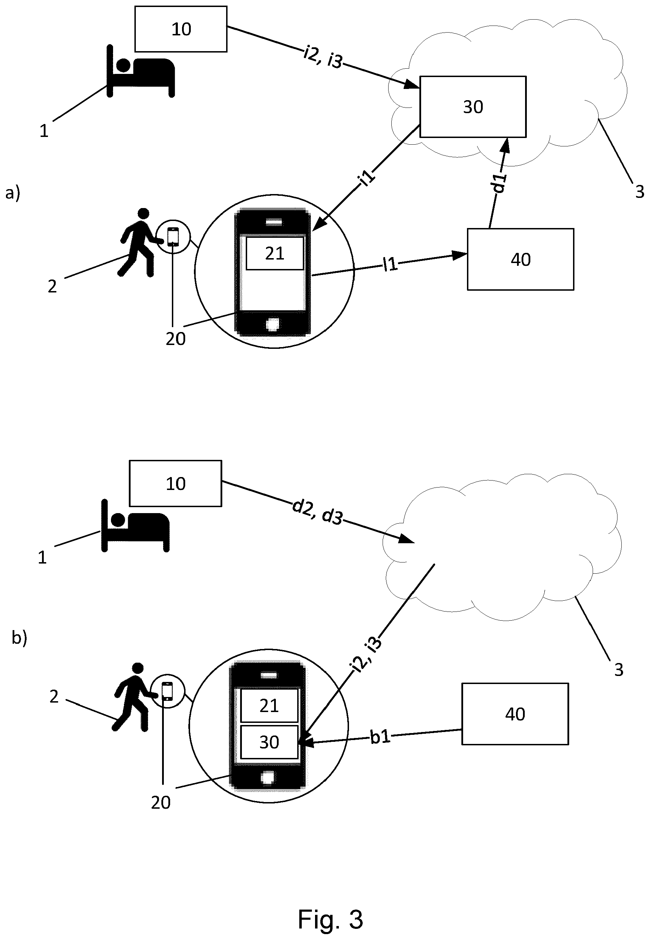

The Figures 3a and 3b illustrate a system for signalling a care need to a user 2 according to two alternatively preferred embodiments of the disclosure. Both embodiments comprise a wearable sensor 10 attached to an incontinence material of a subject 1. They further comprise a device 20, particularly a handheld device 20, held by a user 2. Additionally a network 3 and localisation means 40 are included in both embodiments. The embodiments also both comprise a controller 30, which is configured to perform the above described method.

In the embodiment shown in Figure 3a, the controller 30 is established in the network 3, particularly in a cloud server of the network 3. The wearable sensor 10 detects sensor data and location data associated with the subject 1 and based on this data, sensor information i2 and first location information i3 are determined and send to the controller 30. The sensor information i2 and first location information i3 may be determined by the wearable sensor 10 itself or by an intermediary edge computer which functions as security gate and pre-processing unit and is not shown in Figure 3a.

The localisation means 40 comprise a plurality of transmitters configured to communicate via wired or wireless communication signals, particularly via BLE signals. The device 20 sends localisation signals l1 indicative of a location of the device to the localisation means 40. The localisation signals l1 contain first identification data of the device 20. The controller receives data d1 indicative of the localisation signals 11. The data d1 contains the first identification data, second identification data of the transmitters and signal strengths of the localisation signals 11. This allows the controller 30 to determine where the transmitters are located and based on the signal strengths how distant the device 20 is from them. By means of triangulation, for example, the controller 30 then determines location information of the device 30, which correspond to the second location information.

Furthermore, the controller 30 generates output information i1 using the method described above and sends it to the device 20 of the one user 2. The device 20 then displays the output information i1 to the user 2 using output means 21, such as a screen. The output means 21 display the output information i1 in such a way that a prioritization of the care needs is clearly recognizable, for example as a list, where the prioritized subject information are on top.

In the embodiment shown in Figure 3b, the controller 30 is established in the device 20. The wearable sensor 10 detects sensor data d2 and location data d3 associated with the subject 1 and sends the data d2, d3 to the network, particularly to a cloud server of the network. Based on this data, sensor information i2 and first location information i3 are determined and send to the controller 30. The sensor information i2 and first location information i3 are determined by the cloud server of the network 3 but may also be determined by controller 30 itself which is not shown in Figure 3b.

The localisation means 40 in this embodiment comprise a plurality of beacons configured to send beacon signals b1 to the device 20. A beacon signal b1 contains location data of the sending beacon. By means of the beacon signals b1, the controller 30 can thus determine the locations of several beacons and determine the distances of the device to the beacons on the basis of the strength of the signals b1. By means of triangulation, for example, the controller 30 then determines location information of the device 30, which correspond to the second location information.

Furthermore, the controller 30 generates output information i1 using the method described above on the basis of the received sensor information i2, first location information i3 and the determined second location information. The controller 30 then displays the output information i1 to the user 2 using the output means 21. The output means 21 display the output information i1 in such a way that a prioritization of the care needs is clearly recognizable, for example as a list, where the prioritized subject information are in a red colour.

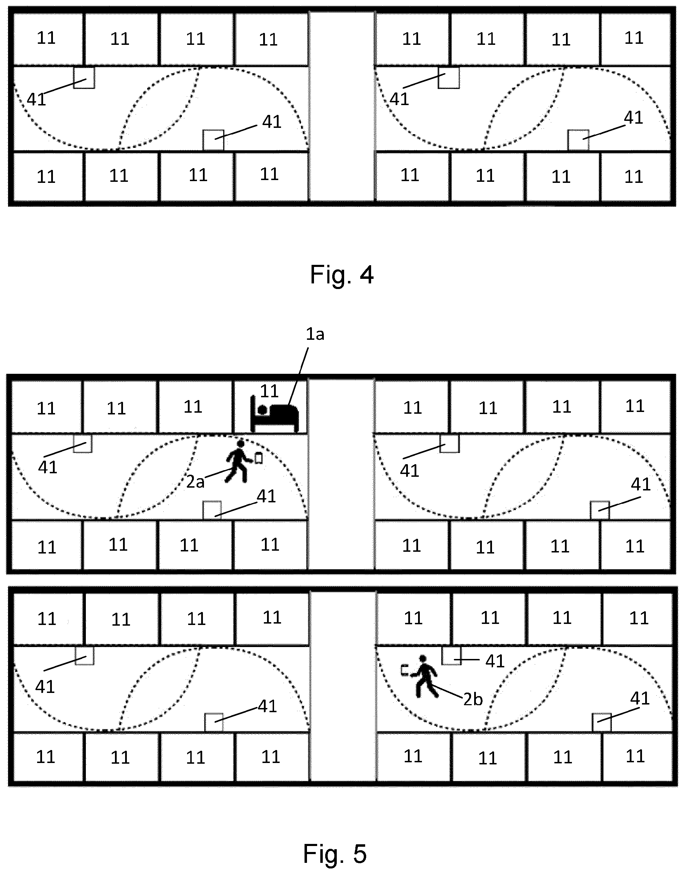

Figure 4 illustrates an exemplary environment, particularly one floor of a nursing home, in which the method according to a preferred embodiment is applied. In other words, a place of use of the system according to the disclosure is illustrated. The exemplary floor of the nursing home has two stations, each of which has eight rooms 11. Two nodes 41 are installed in the corridors of each of the stations. The nodes 41 belong to the localisation means 40 of the system according to the disclosure and are designed here in particular as beacons. The nodes 41 transmit beacon signals b1 within a range represented by the dashed semicircles.

Figure 5 illustrates an exemplary use case of the method according to a preferred embodiment of the disclosure. The exemplary place of use consists of two floors with two stations each, which are structured like the stations shown in Figure 4. In the situation shown, a care need was determined for a first subject 1a. In the process, sensor information i2 and first location information i3 were determined by a wearable sensor 10 worn by the first subject 1a and a controller 30 determined a care need of the subject 1a on this basis. In particular, it was determined that the first subject 1a is the subject with the highest priority care need among all subjects 1 in the place of use. By means of the nodes 41, the users 2a and 2b can be localised. For this purpose, the devices 20 of users 2a and 2b receive the beacon signals b1 of the nodes 41 in whose range they are located. The controller 30 determines, for example, in which station the nodes 41 that sent the received beacon signals b1 are located. Thus, the controller 30 determines the station on which the subject 1a is located as second location information for the first user 2a. The second user 2b is determined as being on another station. Based on the second location information, the controller 30 selects the first user 2a as the recipient of the output information i1, since he or she is the closest to the subject 1 a. The output information i1 is thus generated for the first user 2a, his device 20 outputs the information i1 and the nearest user 2a is sent to the subject with the highest care need 1a.

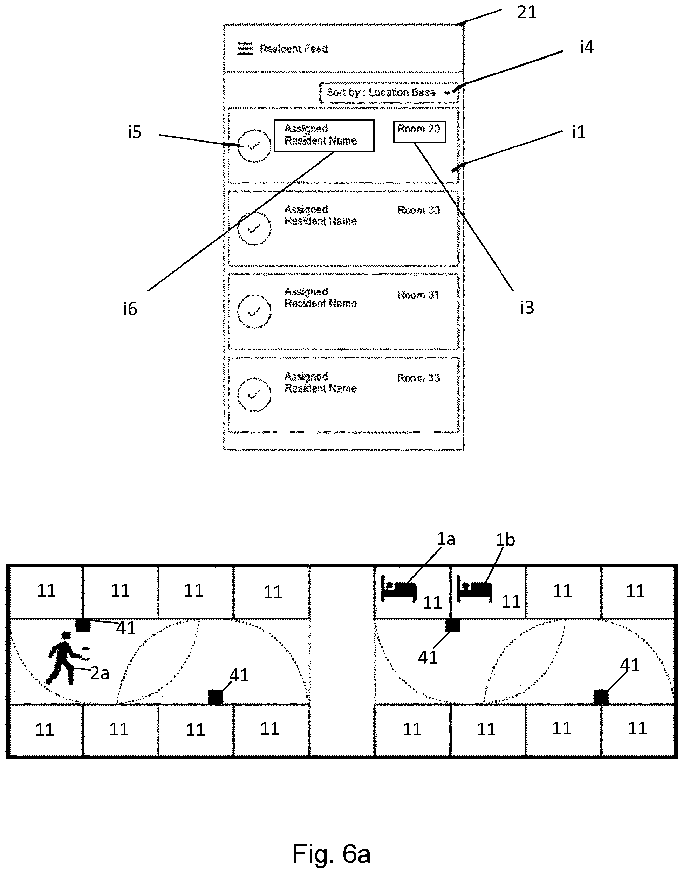

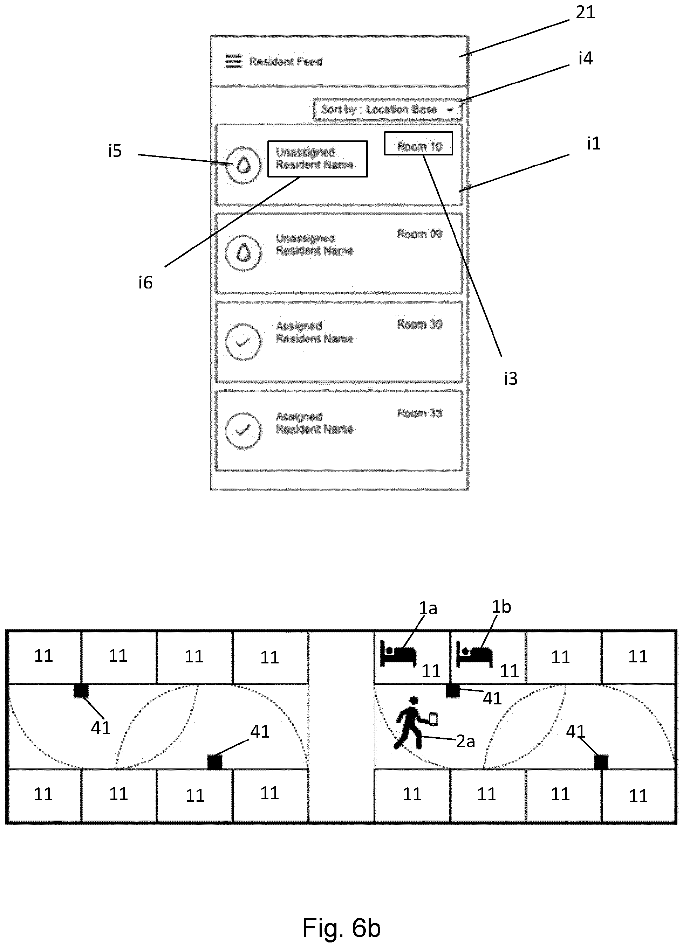

Figure 6a and 6b illustrate another exemplary use case of the method according to a preferred embodiment of the disclosure. The exemplary nursing home in which the method according to the disclosure is applied again has one floor with two stations, as in the example shown in Figure 4. Above the representations of the nursing home, the output means 21 belonging to the device 20 of the user 2a are shown in Figures 6a and 6b respectively. The output means 21 display the output information i1 that is generated for the user 2a in each of the situations shown below.

In the situation shown in Figure 6a, the user 2a is located on a first station and care needs i5 of two subjects 1a and 1b are determined, wherein the subjects 1a and 1b are located on a second station. In this exemplary case, however, the responsibility of user 2a also influences the selection and prioritisation of the subjects 1 that are taken into account in the output information i1. Here, user 2a is responsible for the first station on which he or she is currently located. The output means 21 therefore display output information i1 based on the subjects 1 at his or her station. For each subject assigned to the user 2a, personal information i6 such as the subject's name, first localisation information i3, in particular the room number and a determined care need i5 are displayed. In this example, the care need i5 is displayed in particular as a pictorial representation. A tick in this context means that there is no care need i5 to be treated. In this example, sorting information i4 indicate which information is primarily taken into account for prioritising the care information. In this case, the location plays an overriding role in the prioritisation.

Because there is currently no care need i5 to be treated in his or her station, user 2a now checks on the other station. Figure 6b shows the situation in which the user 2a is now on the other station. Due to the localisation on this station, output information i1 about the subjects 1a and 1b are now displayed to the user 2a by way of the output means 21. In particular, the care needs i5, personal information i6 and first location information i3 of subjects 1a and 1b are displayed. Although the user is not responsible for subjects 1a and 1b, as can be seen from the additionally displayed information "unassigned", he or she is now closest to subjects 1a and 1b. The pictorial representation of the care needs i5 represents them with a drop, which indicates a filled incontinence material. The information of subject 1a in particular are shown here above them of subject 1b. This is because in addition to the identical care need i5 of both subjects 1a and 1b, it was determined by means of a timer that subject 1a has already been affected by the care need for a longer period of time. Therefore, the first subject 1a is displayed with priority in order to suggest to the user 2a that this subject's 1a care need i5 should be treated first.

### Reference list

- 1: subject
- 1a: first subject
- 1b: second subject
- 2: user
- 2a: first user
- 2b: second user
- 3: network
- 10: wearable sensor
- 11: room
- 20: device
- 21: output means
- 30: controller
- 40: localisation means
- 41: node
- b1: beacon signal
- d1: data
- d2: sensor data
- d3: location data
- i1: output information
- i2: sensor information
- i3: first location information
- i4: sorting information
- i5: care need
- i6: personal information
- l1: localisation signal
- S1: first step
- S2: second step
- S3: third step
- S4: forth step

## Claims

1. Method for signalling a care need (i5) to a user (2) by a controller (30) of an electronic device, the method comprising the steps of:
obtaining (S1) sensor information (i2) and first location information (i3) related to at least one subject (1), the sensor information (i2) being indicative of a care need (i5) of the at least one subject (1);
obtaining (S2) second location information related to at least one user (2);
determining (S3) at least one subject (1) in need of care based on the sensor information (i2); and
generating (S4) output information (i1) for one of the at least one user (2), the output information (i1) being based on the care needs (i5) and the first location information (i3) of the at least one subject (1) in need of care and on the second location information.

2. Method according to claim 1, wherein sensor information (i2) and first location information (i3) of a plurality of subjects (1) are obtained and wherein the output information (i1) is related to one of the at least one subject (1) that is determined having a shortest distance to the one user (2) and/or a highest care need (i5).

3. Method according to claim 1 or 2, wherein second location information of a plurality of users (2) are obtained and wherein the one user (2) is determined based on a distance to the at least one subject (1), qualification information and/or capacity information of the plurality of users (2).

4. Method according to claim 2, wherein a plurality of care information (i6) is generated based on the care needs (i5) and the first location information (i3) of the plurality of subjects (1) and prioritized based on the care needs (i5), and wherein the highest priority care information (i6) indicate the highest care need (i5).

5. Method according to claims 3 and 4, wherein a plurality of user information is generated and prioritized based on the qualification information, the capacity information and/or the second location information, wherein the one user (2) has the highest priority user information.

6. Method according to claim 5, wherein a qualification information indicating a qualification for a plurality of care needs (i5), a capacity information indicating a high capacity and/or a second location information indicating a short distance to the at least one subject (1) determines a high priority user information.

7. Method according to claim 4 to 6, wherein the second location information indicate predicted locations of the plurality of users (2).

8. Method according to any one of the preceding claims, wherein the sensor information (i2) comprises at least one of a temperature, a humidity, an acceleration, a gas presence, an orientation and/or a sound level.

9. System for signalling a care need (i5) to a user (2), the system comprising:
a controller (30) configured to perform a method according to any one of claims 1 to 8;
at least one wearable sensor (10) associated with at least one subject (1) and configured to detect first location information (i3) of the at least one subject (1) and sensor information (i2) indicating a care need (i5) of the at least one subject (1);
at least one device (20) associated with at least one user (2) and configured to display the output information (i1) to the at least one user (2); and
localisation means (40) configured to perform a localisation of the at least one device (20).

10. System according to claim 9, wherein the controller (30) is established in a cloud server, the localisation means (40) comprising a plurality of transmitters configured to communicate with the at least one device (20) and the cloud server,
wherein the at least one device (20) is further configured to send localisation signals (11) to the plurality of transmitters, and
the cloud server is configured to receive data (d1) indicative of the localisation signals (11) from the transmitters and to determine second location information of the at least one device (20) based on the data (d1).

11. System according to claim 9, wherein the controller (30) is established in the device (20),
the localisation means (40) comprising a plurality of beacons configured to send beacon signals (b1) to the at least one device (20), and
wherein the at least one device (20) is further configured to receive the beacon signals and to determine second location information of the at least one device (20) based on the beacon signals.

12. System according to any one of the claims 9 to 11, wherein the at least one wearable sensor (10) is configured to detect at least one of a temperature, a humidity, an acceleration, a gas presence, an orientation and/or a sound level.

13. Controller (30) for signalling a care need (i5) to a user (2), the controller (30) configured to perform a method according to any one of the claims 1 to 8.

14. Device (20) for signalling a care need (i5) to a user (2), the device (20) comprising:
a controller (30) configured to perform a method according to any one of the claims 1 to 8; and
output means (21) configured to display the output information (i1) to at least one user (2).

15. Computer program comprising instructions which, when the program is executed by a computer, cause the computer to carry out a method according to any one of the claims 1 to 8.
